# EUROPEAN PATENT SPECIFICATION

(11) **EP 1 651 590 B1**
(45) Date of publication and mention of the grant of the patent: **30.07.2008**
(21) Application number: 04743737.1
(22) Date of filing: 19.07.2004
(51) Int. Cl.: C07C 209/00

(54) **PROCESS FOR THE PREPARATION OF AN IMINE INTERMEDIATE**
VERFAHREN ZUR HERSTELLUNG EINES IMINZWISCHENPRODUKTS
PROCEDE DE PREPARATION D'UN PRODUIT INTERMEDIAIRE IMINE

(30) Priority: 21.07.2003 HU 0302275
(43) Date of publication of application: 03.05.2006
(73) Proprietor: EGIS Gyógyszergyár Nyilvánosan Müködõ Részvénytársaság, 1106 Budapest (HU)
(72) Inventor: BARK CZY, József, H-1016 Budapest (HU); K TAY NAGY, Péter;, H-2600 Vác (HU); SIMIG, Gyula, H-1126 Budapest (HU); J KFALVI, Elemér, H-1161 Budapest (HU); GREGORN BOROS, Livia, H-2141 Csömör (HU); KRASZNAI, György, H-1172 Budapest (HU); VERECZKEYN DON TH, Györgyi, H-1034 Budapest (HU); NAGY, Kálmán, H-1025 Budapest (HU); M METH, Norbert, H-9400 Sopron (HU)
(74) Representative: Hirsch & Associés
(86) International application number: PCT/HU2004/000078
(87) International publication number: WO 2005/007611

(56) References cited:
- EP-A- 1 059 287
- US-A- 4 536 518
- JAESOOK YUN ET AL.: "EFFICIENT KINETIC RESOLUTION IN THE ASYMMETRIC HYDROSILYLATION OF IMINES OF 3-SUBSTITUTED INDANONES AND 4-SUBSTITUTED TETRALONES" J.ORG.CHEM. 65, 2000, pages 767-774, XP001204903

## Description

### BACKGROUND OF THE INVENTION

The invention relates to a new and improved process for the preparation of a ketimine compound which is a useful pharmaceutical intermediate. More particularly, the invention is directed to a new and improved process for the preparation of [4(S,R)-(3,4-dichlorophenyl)-3,4-dihydro-1(2H)-naphthalene-1-ylidene]-methyl-amine of the Formula

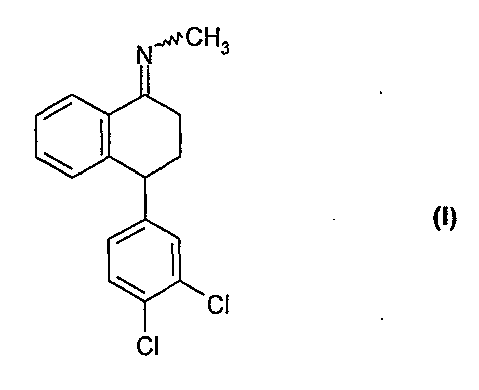

The compound of the Formula I is a valuable intermediate which can be used in pharmaceutical industry by the preparation of cis-(1S)(4S)-N-methyl-4-(3,4-dichlorophenyl)-1,2,3,4-tetrahydro-1-naphthalene-amine-hydrochloride (INN sertraline) being a highly effective antidepressant.

### STATE OF THE ART

The preparation of the compound of the Formula I was first described in US 4,536,518. According to this US patent 4-(3,4-dichlorophenyl)-3,4-dihydro-1(2H)-naphthalene-1-one (tetralone) of the Formula 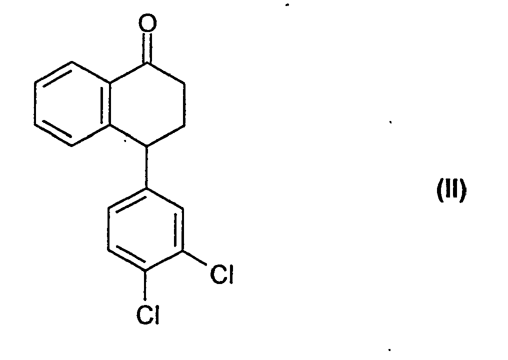 is coupled with methyl-amine in the presence of titan tetrachloride. Methyl-amine is used in a six-fold molar excess and the methyl-amine chlorohydrate and titan dioxide formed is removed by filtration from the reaction mixture. The disadvantage of this process is that titan tetrachloride is a highly corrosive product difficult to handle.

According to US 4,855,500 the reaction is carried out in an aprotic solvent in the presence of a molecular sieve under pressure. The reported yield is 87 %. However, this process is accompanied by the drawback that molecular sieve is very expensive and the reaction is rather slow. For the reason stated above the above process which uses a molecular sieve is unsuitable for the economical manufacture of the desired compound.

According to EP 1,047,666 tetralone is reacted with methyl-amine in a protic solvent at a temperature between 50°C and 110°C. Although high temperature and pressure are used, the conversion of the reaction is not higher than 95-98 %. The significant drawback of the process is that the reaction must be carried out in a special pressure-proof apparatus.

According to WO 00/26181 the reaction is carried out in a protic solvent, in the presence of a catalyst containing sulfonic acid or zeolite or silicate, in the presence of a dehydrating agent. The yield of the [4(S,R)-(3,4-dichlorophenyl)-3,4-dihydro-1(2H)-naphthalene-1-ylidene]-methyl-amine obtained is between 66 % and 86 %.

According to the process described in WO 01/36377 a mixture containing 75-95 % of 4-(3,4-dichlorophenyl)-3,4-dihydro-1(2H)-naphthalene-1-one and 25-5 % of 4-(2,3-dichlorophenyl)-3,4-dihydro-2H-naphthalene-1-one in a solvent is reacted with methyl-amine in the presence of a catalyst which contains sulfonic acid or zeolite or silicate or in the presence of a dehydrating agent under pressure at a temperature between 50°C and 70°C. A large number of solvents is enumerated. The product is purified by crystallization and the compound [4(S,R)-(3,4-dichlorophenyl)-3,4-dihydro-1(2H)-naphthalene-1-ylidene]-methyl-amine is obtained in a purity above 99 %.

According to the process described in WO 02/096860 [4(S,R)-(3,4-dichlorophenyl)-3,4-dihydro-1(2H)-naphthalene-1-ylidene]-methyl-amine is prepared by reacting 4-(3,4-dichlorophenyl)-3,4-dihydro-1(2H)-naphthalene-1-one with methyl-amine is an amide-containing solvent under the conditions of acid catalysis. Under such reaction conditions the reaction takes place rather slowly and requires 10 hours.

According to HU 98/01024 4-(3,4-dichlorophenyl)-3,4-dihydro-1(2H)-naphthalene-1-one is reacted with methyl-amine in a lower alkanol to yield [4(S,R)-(3,4-dichlorophenyl)-3,4-dihydro-1(2H)-naphthalene-1-ylidene]-methyl-amine. HU 98/01025 relates to a reduction process whereby [4(S,R)-(3,4-dichlorophenyl)-3,4-dihydro-1(2H)-naphthalene-1-ylidene]-methyl-amine is reduced to N-methyl-4-(3,4-dichlorophenyl)-1,2,3,4-tetrahydro-1-naphthalene-amine preferably in an ether type solvent, particularly in tetrahydrofurane.

### SUMMARY OF THE INVENTION

The object of the invention is to eliminate the above disadvantages of the known processes and to provide an industrial scale new process for the preparation of [4(S,R)-(3,4-dichlorophenyl)-3,4-dihydro-1(2H)-naphthalene-1-ylidene]-methyl-amine of the Formula I.

The above object is achieved by the process of the present invention.

According to the present invention there is provided a process for the preparation of [4(S,R)-(3,4-dichlorophenyl)-3,4-dihydro-1(2H)-naphthalene-1-ylidene]-methyl-amine of the Formula I by reacting 4-(3,4-dichlorophenyl)-3,4-dihydro-1(2H)-naphthalene-1-one of the Formula II with monomethyl amine which comprises carrying out the reaction in the presence of thionyl chloride in an ether-type solvent.

### DETAILED DESCRIPTION OF THE INVENTION

The present invention is based on the surprising recognition that if the reaction is carried out without using an alcohol-type solvent, titan tetrachloride and molecular sieve as suggested according to the known methods, the compound of the Formula I can be prepared with a very short reaction time and in an excellent yield of 99.5 % by carrying out the reaction 4-(3,4-dichlorophenyl)-3,4-dihydro-1(2H)-naphthalene-1-one of the Formula II and monomethyl amine in the presence of thionyl chloride in an ether-type solvent.

As ether type solvent preferably tetrahydrofurane, dioxane, diethyl ether, diisopropyl ether or methyl-tertiary butyl ether, particularly tetrahydrofurane can be used.

The reaction is carried out at a temperature between -30°C and +40°C, preferably between -10°C and +25°C.

The reaction can be performed under atmospheric pressure without heating. The reaction time is short even under such mild reaction condition and takes 0.5-5 hours, preferably 1-3 hours.

Monomethyl amine is used in an excess related to the compound of the Formula II, namely generally in a 5-15 molar excess, preferably 10-12 molar excess. Thionyl chloride is used in an amount of 1.1-5 mole, preferably 1.5-2 mole, related to 1 mole of the compound of the Formula II.

According to a form of realization of the present invention there is provided a process for the preparation of a mixture of the (+)- and (-)-enantiomers of the compound of the Formula I enriched in the (+)-enantiomer which comprises using as starting material a mixture of the (+)- and (-)-enantiomers of the compound of the Formula II enriched in the (+)-enantiomer.

The [4(S,R)-(3,4-dichlorophenyl)-3,4-dihydro-1(2H)-naphthalene-1-ylidene]-methyl-amine of the Formula I thus obtained can be converted into (+/-)-cis-N-methyl-4-(3,4-dichlorophenyl)-1,2,3,4-tetrahydro-1-naphthalene-amine (raceme sertraline) if desired by catalytic hydrogenation.

According to a further aspect of the present invention there is provided a process for the preparation of (+/-)cis-N-methyl-4-(3,4-dichlorophenyl)-1,2,3,4-tetrahydro-1-naphthalene-amine which comprises reacting 4-(3,4-dichlorophenyl)-3,4-dihydro-1(2H)-naphthalene-1-one of the Formula II with monomethyl amine in the presence of thionyl chloride in an ether-type solvent, and thereafter subjecting the [4(S,R)-(3,4-dichlorophenyl)-3,4-dihydro-1(2H)-naphthalene-1-ylidene]-methyl-amine of the Formula I thus formed to catalytic hydrogenation in the same solvent.

As ether-type solvent preferably tetrahydrofurane, dioxane, diethyl ether, diisopropyl ether or methyl-tertiary butyl ether, particularly tetrahydrofurane can be used.

Catalytic hydrogenation can be carried out preferably in the presence of palladium-charcoal catalyst. One can proceed preferably under atmospheric pressure at room temperature.

The racemic sertraline thus obtained can be optionally converted into a pharmaceutically acceptable acid addition salt thereof, preferably into the hydrochloride.

According to a further aspect of the present invention there is provided a process for the preparation of cis-(1S)(4S)-N-methyl-4-(3,4-dichlorophenyl)-1,2,3,4-tetrahydro-1-naphthalene-amine and pharmaceutically acceptable acid addition salts thereof, preferably the hydrochloride, which comprises reacting 4-(3,4-dichlorophenyl)-3,4-dihydro-1(2H)-naphthalene-1-one of the Formula II with monomethyl amine in the presence of thionyl chloride in an ether-type solvent, subjecting the [4(S,R)-(3,4-dichlorophenyl)-3,4-dihydro-1(2H)-naphthalene-1-ylidene]-methyl-amine of the Formula I thus obtained to catalytic hydrogenation in the same solvent, and thereafter subjecting the (+/-)cis-N-methyl-4-(3,4-dichlorophenyl)-1,2,3,4-tetrahydro-1-naphthalene-amine thus obtained to resolution and if desired converting the product into a pharmaceutically acceptable acid addition salt, preferably the hydrochloride thereof.

As ether-type solvent preferably tetrahydrofurane, dioxane, diethyl ether, diisopropyl ether or methyl-tertiary butyl ether, particularly tetrahydrofurane can be used.

Resolution of racemic sertraline is carried out in a known manner by a process described in prior art preferably by using optically active amygdalic acid.

The significant advantage of the above form of realization of the process of the present invention is that the compound of the Formula I prepared by the present invention can be directly converted into racemic sertraline by catalytic hydrogenation performed without solvent change in the same solvent, in the mixture obtained by the preparation thereof, thereafter the pharmaceutical active ingredient cis-(1S)(4S)-N-methyl-4-(3,4-dichlorophenyl)-1,2,3,4-tetrahydro-1-naphthalene-amine-hydrochloride can be prepared by resolution and salt formation carried out in a manner known per se.

Compared to the processes described in prior art the process of the present invention shows the following advantages:
● the formation of the ketimine of the Formula I and the catalytic reduction can be carried out in the same solvent;
● the reaction time is extremely short and this presents a valuable technological advantage from the point of view of capacity;
● the reaction can be carried out without using high pressure and high temperature;
● the conversion and the yield are extremely high;
● a product of high purity is obtained; and
● the process can be performed in a simple manner under industrial scale too.

Further details of the present invention are to be found in the following Examples without limiting the scope of protection to said Examples.

### Example 1

### Process for the preparation of [4(S,R)-(3,4-dichlorophenyl)-3,4-dihydro-1(2H)-naphthalene-1-ylidene]-methyl-amine

9 g (30.8 mM) of 4-(3,4-dichlorophenyl)-3,4-dihydro-1(2H)-naphthalene-1-one are dissolved in 140 ml of tetrahydrofurane. The solution is cooled to -10°C and 12.6 g (400 mM) of gaseous methyl amine are introduced. To the reaction mixture at -10°C 4.2 ml (60 mM) of thionyl chloride are added under stirring. The addition having been completed the reaction mixture is stirred at room temperature. The reaction is monitored by GC. The reaction mixture is worked up when the amount of the starting material is decreased below 0.3 %. The tetrahydrofurane phase is separated from the precipitated methyl amine hydrochloride, evaporated and the residue is suspended in 20 ml of cold methanol. The crystals obtained are dried under an infra lamp. Thus 8.7 g of the desired compound are obtained, yield 93 %. Mp.: 147-148°C.
IR (KBr): 1625, 1468, 1056, 764.
¹H-NMR (CDCl₃, i400): 8.20 (1H, dd, J2=7.3 Hz, J2=1.9 Hz), 7.3 m (2H), 7.19 (1H, d, J=2.0 Hz), 6.89 m (2H), 4.15 (1H, dd, J1=4.5 Hz, J2=6.8 Hz), 3.17 s (3H), 2.52 m (2H), 2.28 m (1H), 2.13 m (1H).

### Example 2

### Preparation of (+/-)cis-N-methyl-4-(3,4-dichlorophenyl)-1,2,3,4-tetrahydro-1-naphthalene-amine

9 g (30.8 mM) of 4-(3,4-dichlorophenyl)-3,4-dihydro-1(2H)-naphthalene-1-one are dissolved in 140 ml of tetrahydrofurane. The solution is cooled to -10°C and 12.6 g (400 mM) of gaseous methyl amine are introduced. To the reaction mixture 4.2 ml (60 nM) of thionyl chloride are added at -10°C under stirring. The addition having been completed the reaction mixture is stirred at room temperature. The reaction is monitored by GC. The reaction mixture is worked up when the amount of the starting material is decreased below 0.3 %. The tetrahydrofurane phase is made free of methyl amine by partial evaporation: To the residual tetrahydrofurane solution 6.5 ml of a 1:1 mixture of an aqueous solution of 52 ml of potassium chloride and methanol and thereafter 1 g of a palladium-charcoal catalyst (8 % Pd, 28 % C, 64 % H₂O) are added. The reaction mixture is flushed with nitrogen under stirring and thereafter hydrogenated under atmospheric pressure at room temperature. Hydrogenation is continued until the [4(S,R)-(3,4-dichlorophenyl)-3,4-dihydro-1(2H)-naphthalene-1-ylidene]-methyl-amine is consumed. After the termination of hydrogenation the reaction mixture is filtered and the filtrate is acidified with concentrated aqueous hydrochloric acid at room temperature. The precipitated crystals are filtered. Thus 8.6 g of the desired compound are obtained, yield 82 %. M.p.: 280-283 °C.
IR (KBr): 3340, 3021, 1469, 1396, 878.
¹H-NMR (CDCl₃, i400): 10.03 (1H, s), 9.93 (1H, s), 7.77 (1H, d, J=7.4 Hz), 7.41 (1H, d, 1.9 Hz), 7.36 (1H, d, J=8.2 Hz), 7.2 (3H, m), 6.87 (1H, d, J=7.5 Hz), 4.3 (1H, kv, J=4.9 Hz), 4.00 (1H, dd, J1=5.4 Hz, J2=8.4 Hz), 2.58 (3H, t, J=5.4 Hz), 2.3 (2H, m), 2.1 (2H, m).

### Example 3

### Preparation of cis-(1S)(4S)-N-methyl-4-(3,4-dichlorophenyl)-1,2,3,4-tetrahydro-1-naphthalene-amine-hydrochloride (sertraline hydrochloride)

From (+/-)cis-N-methyl-4-(3,4-dichlorophenyl)-1,2,3,4-tetrahydro-1-naphthalene-amine-hydrochloride the base is set free and thereafter subjected to resolution with amygdalic acid. The sertraline base is set free and converted into the hydrochloride. The desired compound is obtained with a yield of 46 %. Mp: 243-245°C.
Optical rotation: [a]_{D}²² = +37,7 (c=2, methanol).
IR (KBr): 3340, 3021, 1469, 1396, 878.
¹H-NMR (CDCl₃, i400): 10.03 (1H, s), 9.93 (1H, s), 7.77 (1H, d, J=7.4 Hz), 7.41 (1H, d, 1.9 Hz), 7.36 (1H, d, J=8.2 Hz), 7.2 (3H, m), 6.87 (1H, d, J=7.5 Hz), 4.3 (1H, kv, J=4.9 Hz), 4.00 (1H, dd, J1=5.4 Hz, J2=8.4 Hz), 2.58 (3H, t, J=5.4 Hz), 2.3 (2H, m), 2.1 (2H, m).

## Claims

1. Process for the preparation of [4(S,R)-(3,4-dichlorophenyl)-3,4-dihydro-1(2H)-naphthalene-1-ylidene]-methyl-amine of the Formula 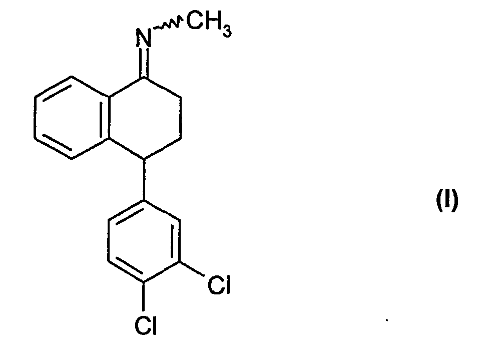 by reacting 4-(3,4-dichlorophenyl)-3,4-dihydro-1(2H)-naphthalene-1-one of the Formula 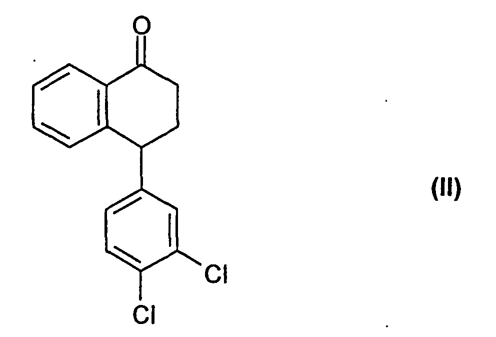 with monomethyl amine which comprises carrying out the reaction in the presence of thionyl chloride in an ether-type solvent.

2. Process according to Claim 1 which comprises using as ether-type solvent tetrahydrofurane, dioxane, diethyl ether, diisopropyl ether or methyl-tertiary butyl ether.

3. Process according to Claim 2 which comprises using tetrahydrofurane as ether-type solvent.

4. Process according to any of Claims 1-3 which comprises carrying out the reaction at a temperature between - 30°C and +40°C.

5. Process according to Claim 4 which comprises carrying out the reaction at a temperature between -10°C and +25°C.

6. Process according to Claim 1 wherein the reaction time is between 0.5 hour and 5 hours.

7. Process according to Claim 6 wherein the reaction time is 1-3 hours.

8. Process according to Claim 1 which comprises using thionyl chloride in an amount of 1.1-5 moles, related to 1 mole of the compound of the Formula II.

9. Process according to Claim 8 which comprises using thionyl chloride in an amount of 1.5-2 moles, related to 1 mole of the compound of the Formula II.

10. Process according to any of Claims 1-9 for the preparation of a mixture of the (+)- and (-)-enantiomers of the compound of the Formula I enriched in the (+)-enantiomer which comprises using as starting material a mixture of the (+)- and (-)-enantiomers of the compound of the Formula II enriched in the (+)-enantiomer.

11. Process according to any of Claims 1-10 which comprises convering [4(S,R)-(3,4-dichlorophenyl)-3,4-dihydro-1(2H)-naphthalene-1-ylidene]-methyl-amine of the Formula I into (+/-)cis-N-methyl-4-(3,4-dichlorophenyl)-1,2,3,4-tetrahydro-1-naphthalene-amine (racemic sertraline) by catalytic hydrogenation.

12. Process according to any of Claims 1-11 for the preparation of (+/-)cis-N-methyl-4-(3,4-dichlorophenyl)-1,2,3,4-tetrahydro-1-naphthalene-amine which comprises reacting 4-(3,4-dichlorophenyl)-3,4-dihydro-1(2H)-naphthalene-1-one of the Formula II with monomethyl amine in the presence of thionyl chloride in an ether-type solvent, and thereafter subjecting the [4(S,R)-(3,4-dichlorophenyl)-3,4-dihydro-1(2H)-naphthalene-1-ylidene]-methyl-amine of the Formula I thus formed to catalytic hydrogenation in the same solvent.

13. Process according to Claim 12 which comprises using as ether-type solvent tetrahydrofurane, dioxane, diethyl ether, diisopropyl ether or methyl-tertiary butyl ether.

14. Process according to Claim 13 which comprises using tetrahydrofurane as ether-type solvent.

15. Process according to any of Claims 1-14 for the preparation of cis-(1S)(4S)-N-methyl-4-(3,4-dichlorophenyl)-1,2,3,4-tetrahydro-1-naphthalene-amine and pharmaceutically acceptable acid addition salts, preferably the hydrochloride thereof which comprises reacting 4-(3,4-dichlorophenyl)-3,4-dihydro-1(2H)-naphthalene-1-one of the formula II with monomethyl amine in the presence of thionyl chloride in an ether-type solvent; subjecting the [4(S,R)-(3,4-dichlorophenyl)-3,4-dihydro-1(2H)-naphthalene-1-ylidene]-methyl-amine of the Formula I thus formed to catalytic hydrogenation in the same solvent; subjecting the (+/-)cis-N-methyl-4-(3,4-dichlorophenyl)-1,2,3,4-tetrahydro-1-naphthalene-amine thus obtained to resolution and if desired converting the (+/-)cis-N-methyl-4-(3,4-dichlorophenyl)-1,2,3,4-tetrahydro-1-naphthalene-amine into a pharmaceutically acceptable salt, preferably the hydrochloride thereof.

16. Process according to Claim 15 which comprises using as ether-type solvent tetrahydrofurane, dioxane, diethyl ether, diisopropyl ether or methyl-tertiary butyl ether.

17. Process according to Claim 16 which comprises using tetrahydrofurane as ether-type solvent.

## Patentansprüche

1. Verfahren zur Herstellung von [4(S,R)-(3,4-Dichlorphenyl)-3,4-dihydro-1(2H)-naphthalen-1-yliden]-methylamin mit der Formel 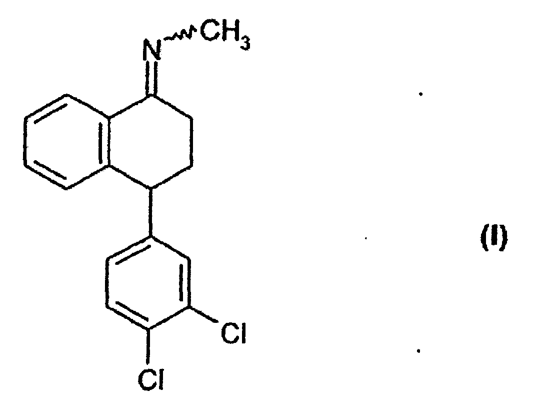 durch das Reagieren von 4-(3,4-Dichlorphenyl)-3,4-dihydro-1(2H)-naphthalen-1-on mit der Formel 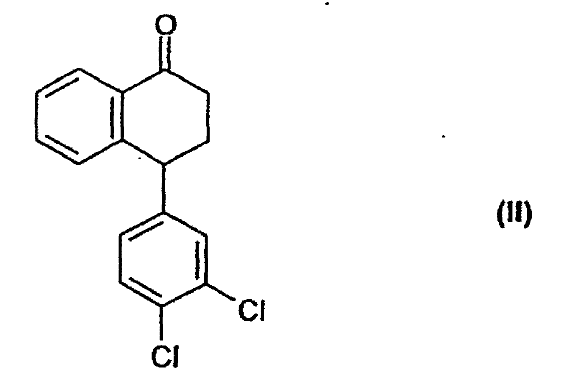 mit Monomethylamin, welches das Durchführen der Reaktion in Gegenwart von Thionylchlorid in einem Lösemittel des Ethertyps umfasst.

2. Verfahren nach Anspruch 1, welches die Verwendung von Tetrahydrofuran, Dioxan, Diethylether, Diisopropylether oder Methyltertiärbutylether als das Lösemittel des Ethertyps umfasst.

3. Verfahren nach Anspruch 2, welches die Verwendung von Tetrahydrofuran als das Lösemittel des Ethertyps umfasst.

4. Verfahren nach einem der Ansprüche 1-3, welches das Durchführen der Reaktion bei einer Temperatur zwischen -30 °C und +40 °C umfasst.

5. Verfahren nach Anspruch 4, welches das Durchführen der Reaktion bei einer Temperatur zwischen -10 °C und +25 °C umfasst.

6. Verfahren nach Anspruch 1, wobei die Reaktionszeit zwischen 0,5 Stunden und 5 Stunden beträgt.

7. Verfahren nach Anspruch 6, wobei die Reaktionszeit 1-3 Stunden beträgt.

8. Verfahren nach Anspruch 1, welches die Verwendung von Thionylchlorid in einer Menge von 1,1-5 Mol in Bezug auf 1 Mol der Verbindung mit der Formel II umfasst.

9. Verfahren nach Anspruch 8, welches die Verwendung von Thionylchlorid in einer Menge von 1,5-2 Mol in Bezug auf 1 Mol der Verbindung mit der Formel II umfasst.

10. Verfahren nach einem der Ansprüche 1-9 für die Herstellung einer Mischung aus den (+)- und (-)-Enantiomeren der Verbindung mit der Formel I angereichert mit dem (+)-Enantiomer, welches die Verwendung einer Mischung aus den (+)- und (-)-Enantiomeren der Verbindung mit der Formel II angereichert mit dem (+)-Enantiomer als Ausgangsmaterial umfasst.

11. Verfahren nach einem der Ansprüche 1-10, welches die Umwandlung des [4(S,R)-(3,4-Dichlorphenyl)-3,4-dihydro-1(2H)-naphthalen-1-yliden]-methylamin mit der Formel I in (+/-)cis-N-Methyl-4-(3,4-dichlorphenyl)-1,2,3,4-tetrahydro-1-naphthalenamin (racemisches Sertralin) durch katalytische Hydrierung umfasst.

12. Verfahren nach einem der Ansprüche 1-11 für die Herstellung von (+/-)cis-N-Methyl-4-(3,4-dichlorphenyl)-1,2,3,4-tetrahydro-1-naphthalenamin, welches das Reagieren von 4-(3,4-Dichlorphenyl)-3,4-dihydro-1(2H)-naphthalen-1-on mit der Formel II mit Monomethylamin in Gegenwart von Thionylchlorid in einem Lösemittel des Ethertyps und danach die katalytische Hydrierung des so gebildeten [4(S,R)-(3,4-Dichlorphenyl)-3,4-dihydro-1(2H)-naphthalen-1-yliden]-methylamin mit der Formel I in dem gleichen Lösemittel umfasst.

13. Verfahren nach Anspruch 12, welches die Verwendung von Tetrahydrofuran, Dioxan, Diethylether, Diisopropylether oder Methyltertiärbutylether als das Lösemittel des Ethertyps umfasst.

14. Verfahren nach Anspruch 13, welches die Verwendung von Tetrahydrofuran als das Lösemittel des Ethertyps umfasst.

15. Verfahren nach einem der Ansprüche 1-14 für die Herstellung von cis-(1S)(4S)-N-Methyl-4-(3,4-dichlorphenyl)-1,2,3,4-tetrahydro-1-naphthalenamin und pharmazeutisch akzeptablen Säureadditionssalzen, vorzugsweise das Hydrochlorid davon, welches das Reagieren von 4-(3,4-Dichlorphenyl)-3,4-dihydro-1(2H)-naphthalen-1-on mit der Formel II mit Monomethylamin in Gegenwart von Thionylchlorid in einem Lösemittel des Ethertyps; die katalytische Hydrierung des so gebildeten [4(S,R)-(3,4-Dichlorphenyl)-3,4-dihydro-1(2H)-naphthalen-1-yliden]-methylamin mit der Formel I in dem gleichen Lösemittel; die Auflösung des so gebildeten (+/-)cis-N-Methyl-4-(3,4-dichlorphenyl)-1,2,3,4-tetrahydro-1-naphthalenamin und, falls gewünscht, die Umwandlung des (+/-)cis-N-Methyl-4-(3,4-dichlorphenyl)-1,2,3,4-tetrahydro-l-naphthalenamin in ein pharmazeutisch akzeptables Salz, vorzugsweise das Hydrochlorid davon, umfasst.

16. Verfahren nach Anspruch 15, welches die Verwendung von Tetrahydrofuran, Dioxan, Diethylether, Diisopropylether oder Methyltertiärbutylether als das Lösemittel des Ethertyps umfasst.

17. Verfahren nach Anspruch 16, welches die Verwendung von Tetrahydrofuran als das Lösemittel des Ethertyps umfasst.

## Revendications

1. Procédé de préparation de [4(S,R)-(3,4-dichlorophényl)-3,4-dihydro-1(2H)-naphthalène-1-ylidène]-méthyl-amine de formule 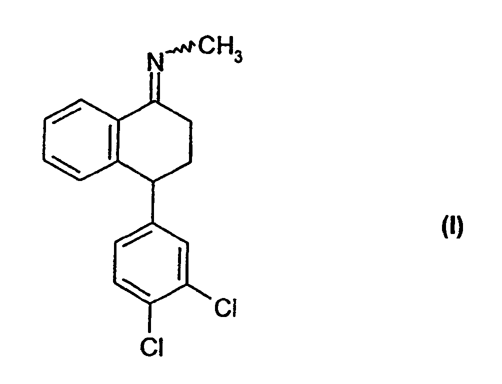 en faisant réagir de la 4-(3,4-dichlorophényl)-3,4-dihydro-1(2H)-naphtalène-1-one de formule 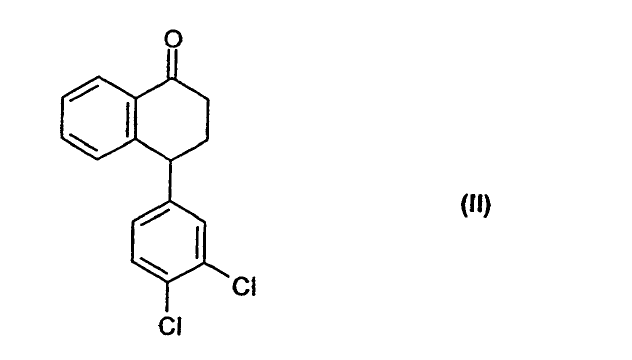 avec de la monométhyl-amine qui comprend l'étape consistant à réaliser la réaction en présence de chlorure de thionyle dans un solvant de type éther.

2. Procédé selon la revendication 1 qui comprend l'utilisation de tétrahydrofuranne, de dioxanne, d'éther diéthylique, d'éther diisopropylique ou d'éther méthyl-tert-butylique en tant que solvant de type éther.

3. Procédé selon la revendication 2 qui comprend l'utilisation de tétrahydrofuranne en tant que solvant de type éther.

4. Procédé selon l'une quelconque des revendications 1 à 3, dans lequel la réaction est réalisée à une température comprise entre -30°C et +40°C.

5. Procédé selon la revendication 4, dans lequel la réaction est réalisée à une température comprise entre -10°C et +25°C.

6. Procédé selon la revendication 1, dans lequel la durée de la réaction est comprise entre 0,5 heure et 5 heures.

7. Procédé selon la revendication 6, dans lequel la durée de la réaction est comprise entre 1 heure et 3 heures.

8. Procédé selon la revendication 1 qui comprend l'utilisation de chlorure de thionyle dans une quantité de 1,1 à 5 moles, par rapport à 1 mole du composé de formule II.

9. Procédé selon la revendication 8 qui comprend l'utilisation de chlorure de thionyle à raison de 1,5 à 2 moles, par rapport à 1 mole du composé de formule II.

10. Procédé selon l'une quelconque des revendications 1 à 9 pour la préparation d'un mélange des énantiomères (+) et (-) du composé de formule I enrichi en énantiomère (+), qui comprend l'utilisation, en tant que matière de départ, d'un mélange des énantiomères (+) et (-) du composé de formule II enrichi en énantiomère (+).

11. Procédé selon l'une quelconque des revendications 1 à 10 qui comprend l'étape consistant à convertir la [4(S,R)-(3,4-dichlorophényl)-3,4-dihydro-1(2H)-naphtalène-1-ylidène]-méthyl-amine de formule I en (+/-)cis-N-méthyl-4-(3,4-dichlorophényl)-1,2,3,4-tétrahydro-1-naphtalène-amine (sertraline racémique) au moyen d'une hydrogénation catalytique.

12. Procédé selon l'une quelconque des revendications 1 à 11 pour la préparation de (+/-) cis-N-méthyl-4-(3,4-dichlorophényl)-1,2,3,4-tétrahydro-1-naphtalène-amine qui comprend les étapes consistant à faire réagir la 4-(3,4-dichlorophényl)-3,4-dihydro-1(2H)-naphthalène-1-one de formule II avec de la monométhyl-amine en présence de chlorure de thionyle dans un solvant de type éther, puis à soumettre la [4(S,R)-(3,4-dichlorophényl)-3,4-dihydro-1(2H)-naphthalène-1-ylidène]-méthyl-amine de formule I ainsi formée à une hydrogénation catalytique dans le même solvant.

13. Procédé selon la revendication 12 qui comprend l'utilisation de tétrahydrofuranne, de dioxanne, d'éther diéthylique, d'éther diisopropylique ou d'éther méthyl-tert-butylique en tant que solvant de type éther.

14. Procédé selon la revendication 13 qui comprend l'utilisation de tétrahydrofuranne en tant que solvant de type éther.

15. Procédé selon l'une quelconque des revendications 1 à 14 pour la préparation de cis-(1S)(4S)-N-méthyl-4-(3,4-dichlorophényl)-1,2,3,4-tétrahydro-1-naphtalène-amine et de sels d'addition d'acide acceptables sur le plan pharmaceutique, de préférence son chlorhydrate, qui comprend les étapes consistant à faire réagir la 4-(3,4-dichlorophényl)-3,4-dihydro-1(2H)-naphtalène-1-one de formule II avec de la monométhyl-amine en présence de chlorure de thionyle dans un solvant de type éther ; soumettre la [4(S,R)-(3,4-dichlorophényl)-3,4-dihydro-1(2H)-naphtalène-1-ylidène]-méthyl-amine de formule I ainsi formée à une hydrogénation catalytique dans le même solvant ; soumettre la (+/-)cis-N-méthyl-4-(3,4-dichlorophényl)-1,2,3,4-tétrahydro-1-naphtalène-amine ainsi obtenue à un dédoublement et si nécessaire convertir la (+/-)cis-N-méthyl-4-(3,4-dichlorophényl)-1,2,3,4-tétrahydro-1-naphtalène-amine en un sel acceptable sur le plan pharmaceutique, de préférence son chlorhydrate.

16. Procédé selon la revendication 15 qui comprend l'utilisation de tétrahydrofuranne, de dioxanne, d'éther diéthylique, d'éther diisopropylique ou d'éther méthyl-tert-butylique en tant que solvant de type éther.

17. Procédé selon la revendication 16 qui comprend l'utilisation de tétrahydrofuranne en tant que solvant de type éther.
